# FASCICULE DE BREVET EUROPEEN

(11) **EP 0 434 581 B1**
(45) Date de publication et mention de la délivrance du brevet: **22.02.1995**
(21) Numéro de dépôt: 90420553.1
(22) Date de dépôt: 18.12.1990
(51) Int. Cl.: B01F 1/00, G01N 13/00

(54) **Cellule de dissolution pour solides et appareil d'étude de la cinétique de dissolution la comportant**
Lösezelle für Feststoffe und Vorrichtung mit einer solchen Zelle zur Untersuchung der Lösekinetik
Solid materials dissolution cell and device containing this cell for determining rate of dissolution

(30) Priorité: 20.12.1989 FR 8917214
(43) Date de publication de la demande: 26.06.1991
(73) Titulaire: SOCIETE PROLABO, 94120 Fontenay-sous-Bois (FR)
(72) Inventeur: Bart, Gilles, F-45450 Donnery (FR); Dequin, Roland, F-45250 Briare (FR); Paturat, F-45250 Briare (FR)
(74) Mandataire: Ahner, Francis

(56) Documents cités:
- EP-A- 0 043 226
- US-A- 3 620 675
- US-A- 3 802 272
- US-A- 4 247 298
- US-A- 4 856 909
- Note pharmacopeé pour 1990 paragraphe V.5.4.A intituleé "Méthode à cellule à flux continu"

## Description

La présente invention concerne une cellule de dissolution pour solides. Elle concerne également un appareil d'étude de la cinétique de dissolution d'un solide qui comporte au moins une telle cellule.

En recherche et dans l'industrie on a souvent besoin de connaître la vitesse à laquelle une matière solide soluble est dissoute par un solvant. Un tel besoin est particulièrement ressenti dans la recherche en pharmacologie et en biologie. Par exemple, en pharmacologie la connaissance de la vitesse à laquelle une matière solide se dissout dans un solvant est nécessaire à la détermination de l'efficacité et des caractéristiques de cette matière solide. Ainsi, la vitesse à laquelle un comprimé, se dissout dans le tube digestif influe sur la vitesse à laquelle les composants actifs du comprimé entreront dans le courant sanguin du malade. Il est donc nécessaire de connaître parfaitement cette vitesse pour définir aussi bien la composition, que la présentation d'un médicament sous forme solide, afin que celui-ci procure l'effet attendu.

Dans l'industrie, la mesure de la vitesse de dissolution d'un comprimé est fréquemment utilisée dans les procédures de contrôle de fabrication.

Un comprimé est généralement composé de deux éléments principaux qui sont le pouvoir actif et le liant qui permet de donner une forme et une tenue mécanique au comprimé, emprisonne le pouvoir actif et le libère progressivement après absorption.

Les méthodes d'études de la cinétique de dissolution d'un comprimé, ou plus généralement d'un solide, sont définies par la commission de pharmacopée européenne dans la note pharmacopée pour 1990 paragraphe V.5.4.A. intitulée "Méthode à cellule à flux continu".

En particulier, la note pharmacopée précise les règles d'étude de la cinétique de dissolution d'un produit solide par la méthode de la cellule à flux continu.

L'appareil proposé est constitué principalement par une cellule, en verre ou en matière plastique, dont la partie utile est un cylindre, à la partie inférieure du cylindre est accolé un cône, percé d'un trou à son sommet pour l'alimentation du solvant. A la partie supérieure du cylindre est adapté un filtre circulaire, par exemple en microfibres de verre, selon toute la section de la partie utile. Afin de répartir uniformément le solvant dans toute la section de la cellule, la partie conique est remplie d'un garnissage par exemple, de billes de verre. Après avoir traversé le filtre, le soluté est évacué en partie haute de la cellule et dirigé vers un système de collecte d'échantillons ou directement vers un appareil d'analyse.

La note pharmacopée précise les caractéristiques dimensionnelles de la partie utile de la cellule et du cône et recommande de balayer la cellule par un flux continu de solvant avec un débit mesuré avec une précision de 5 %.

Pour avoir de bons résultats d'analyse il est nécessaire de retenir toutes les particules de liant présentes dans la cellule, pour cela on utilise des filtres dont la porosité est de quelques microns. Il se produit alors un phénomène de colmatage du filtre qui entraîne une augmentation des pertes de charges du circuit solvant/soluté et par voie de conséquence produit une augmentation de la pression à l'intérieur de la cellule.

Pour limiter ce phénomène il est donc important d'avoir une grande surface de filtration. On a aussi parfois besoin, dans des cas spécifiques de pouvoir augmenter pour une cellule donnée la surface de filtration.

Or ceci est impossible avec une cellule telle que décrite dans la note pharmacopée puisque la surface du filtre est déterminée par la section interne de la cellule.

Or une cellule telle que décrite ci-avant ne permet pas d'introduire de façon aisée une ou plusieurs sondes de mesure dans le milieu liquide de la cellule.

La présente invention concerne précisément une nouvelle cellule de dissolution et un appareil d'étude de la cinétique de dissolution d'un solide comportant une telle cellule, qui évitent ces inconvénients.

Un but de l'invention est une cellule de dissolution pour solides qui permet de disposer d'une surface de filtration plus grande et qui permet en outre de faire varier la surface de filtration de la cellule selon le solide étudié.

Selon l'invention, la cellule de dissolution pour solides est caractérisée en ce qu'elle comprend :
- un récipient (1) fermé à l'extrémité basse (4) et ouvert à l'extrémité haute (5) présentant intérieurement de bas en haut :
   . une zone conique (6) divergente portant des moyens d'entrée de liquide (9) à son sommet, ladite zone contenant un garnissage (10) pour répartir le liquide,
   . une première (7) et une deuxième (8) zones sensiblement cylindrique, la première zone (7) étant d'une section inférieure à celle de la deuxième zone (8), lesdites zones étant réunies par un épaulement (11),
   . la zone conique (6) et la première zone cylindrique (7) formant la chambre de dissolution (24),
- un bouchon (2) destiné à s'adapter de façon étanche à l'extrémité ouverte (5) du récipient (1) et comportant des moyens de sortie (15) du soluté,
caractérisée en ce que l'on peut faire varier la position du bouchon (2) par rapport à l'extrémité ouverte du récipient (1), et en ce que la cellule comprend en outre des moyens de filtration constitués par un filtre tubulaire (20) maintenu entre le bouchon (2) et l'épaulement (11), et placé sur le trajet du soluté entre la chambre de dissolution (24) et les moyens de sortie (15) du soluté.

Le filtre tubulaire peut être réalisé en divers matériaux habituellement utilisés tels que de la céramique poreuse, de la céramique poreuse imprégnée d'acier, des fils d'acier inoxydable. De préférence le filtre tubulaire est formé de microfibres de verre agencées entre elles par tout moyen connu pour constituer un article textile par exemple du genre tissu, nappe, non tissé, tricot.

Avantageusement, le bouchon de la cellule objet de l'invention, comporte des moyens de passage pour introduire au moins une sonde de mesure dans la chambre de dissolution. La sonde de mesure, d'un type connu, permet de suivre l'évolution ou de contrôler divers paramètres du milieu liquide présent dans la cellule tels que température, pH, pression.

Selon un mode de réalisation de la cellule selon l'invention, le bouchon peut s'adapter à l'extrémité ouverte du récipient, extérieurement au récipient. Il peut alors être emmanché de force, de préférence le bouchon est vissé à l'extrémité ouverte du récipient qui est alors pourvue extérieurement à cet effet, d'un pas de vis.

Selon un autre mode de réalisation préférentiel, le bouchon s'adapte à l'extrémité ouverte du récipient, intérieurement au récipient.

Selon ce mode de réalisation le bouchon est inséré de force dans l'extrémité ouverte du récipient et comporte avantageusement de l'extérieur à l'intérieur de la cellule, un corps d'une section sensiblement égale à celle de la deuxième zone cylindrique du récipient et une tête d'une section inférieure à celle du corps et les moyens de sortie du soluté sont constitués par un conduit traversant le corps et débouchant dans l'espace situé entre la tête et la paroi du récipient.

De préférence, le bouchon comporte entre le corps et la tête une zone de collecte constituée par exemple, par une gorge annulaire.

Le bouchon peut être réalisé en divers matériaux qui présentent une bonne inertie chimique afin de ne pas perturber les mesures effectuées. Parmi ces matériaux on peut citer le polytétrafluoroéthylène, les polymères organosiliciques .

Le récipient peut être réalisé en divers matériaux qui présentent, pour les mêmes raisons, une bonne inertie chimique et qui sont de préférences transparents, tels que le verre ou des matières plastiques telles que le polychlorure de vinyle, le polyméthacrylate de méthyle.

Afin que les particules de liant présentes dans la chambre de dissolution ne puissent s'échapper hors de celle-ci vers l'appareil d'analyse il est nécessaire qu'une certaine étanchéité soit réalisée entre le récipient et le filtre et/ou entre le bouchon et le filtre.

L'étanchéité peut être obtenue au moyens de nervures annulaires portées par l'épaulement et le bouchon, le filtre étant maintenu entre le bouchon et l'épaulement par l'intermédiaire de ces nervures. De par la souplesse du matériau constituant le filtre, les nervures pénètrent dans celui-ci lors du montage de la cellule et l'étanchéité de la chambre de dissolution vis-à-vis des particules de liant est assurée.

De préférence, l'étanchéité est réalisée par des moyens d'étanchéité constitués par des joints qui sont le plus souvent des joints toriques.

Le produit solide dont on souhaite déterminer la vitesse de dissolution peut être placé dans la chambre de dissolution directement sur ou dans le lit de billes de verre. Il peut aussi être maintenu par des moyens appropriés tels, par exemple, qu'une nacelle comme décrite dans la note pharmacopée.

Il a également été trouvé un appareil d'étude de la cinétique de dissolution d'un solide qui comporte au moins une cellule de dissolution selon l'invention, à chaque cellule étant associés des moyens de pompage.

Des moyens de pompage qui permettent de délivrer le solvant avec un débit précis et régulier conviennent particulièrement bien.

Avantageusement, l'appareil, objet de l'invention est tel que les moyens de pompage comportent au moins deux pistons montés en opposition déplacés par une came tournant autour d'un axe perpendiculaire à l'axe de déplacement des pistons et des moyens de rappel associes à chaque piston.

La cellule de dissolution d'un solide et l'appareil la comportant, objets de l'invention, peuvent être utilisés chaque fois qu'il est nécessaire de connaître la cinétique de dissolution d'un solide dans un solvant. Ils sont particulièrement destinés à l'étude de la cinétique de dissolution des produits pharmaceutiques sous forme solide tels que les comprimés.

L'invention sera mieux comprise par la description des figures ci-jointes qui illustrent schématiquement, à titre d'exemple, et sans échelle déterminée, divers modes de réalisation de la cellule de dissolution et de l'appareil, objets de l'invention.

La figure 1 est une vue générale en coupe par un plan diamétral, d'un premier mode de réalisation d'une cellule de dissolution ne faisant pas partie de l'invention mais du type général des cellules de l'invention.

La figure 2 est une vue générale en coupe par un plan diamétral, d'un mode de réalisation d'une cellule de dissolution, objet de l'invention.

La figure 3 est une vue partielle en coupe par un plan diamétral, d'un mode de réalisation des moyens d'étanchéité entre le filtre et le bouchon et entre le filtre et le récipient.

La figure 4 est une vue partielle en coupe par un plan diamétral, d'un autre mode de réalisation des moyens de filtration.

La figure 5 est un schéma de principe d'un mode de réalisation des moyens de pompage d'un appareil d'étude de la cinétique de dissolution d'un solide, objet de l'invention.

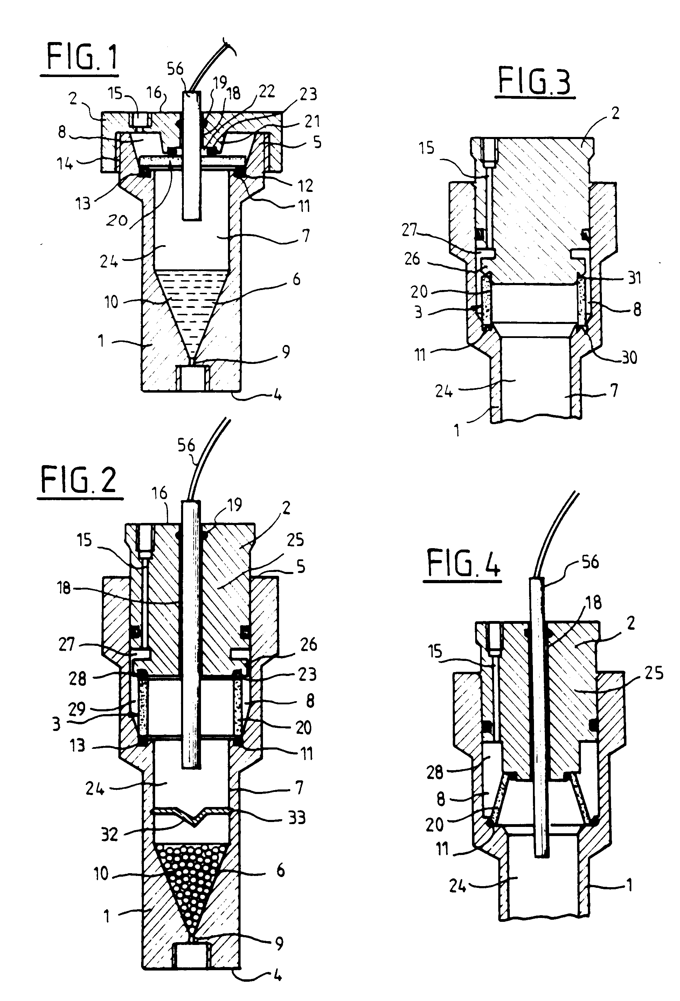

La cellule de dissolution pour solides représentée à la figure 1, comprend un récipient (1), un bouchon (2) et des moyens de filtration (20). Le récipient (1) est fermé à l'extrémité basse (4) et ouvert à l'extrémité haute (5). Le récipient (1) présente intérieurement de bas en haut une zone conique (6), une première zone cylindrique (7) et une deuxième zone cylindrique (8).

La zone conique (6) du récipient (1) est divergente de bas en haut, elle présente au sommet du cône des moyens d'entrée du liquide constitués par un conduit (9). Au voisinage de l'extérieur du récipient (1), le conduit (9) est avantageusement taraudé pour permettre l'adaptation aisée, au moyen de raccord, du conduit d'alimentation de liquide.

La zone conique (6) du récipient (1) contient un garnissage (10) destiné à répartir uniformément le liquide dans toute la section du récipient. Ce garnissage (10) est le plus souvent constitué par des billes de verre.

Le produit solide peut être placé directement sur ou dans le garnissage (10) de billes de verre.

Au dessus de la zone conique (6) le récipient (1) présente la première zone cylindrique (7) surmontée de la deuxième zone cylindrique (8). La première zone cylindrique (7) a un diamètre égal au diamètre de base du cône et la deuxième zone cylindrique (8) a un diamètre supérieur à celui de la première zone cylindrique (7). De par la différence de dimensions de leurs diamètres la première zone cylindrique (7) a une section inférieure à la section de la deuxième zone cylindrique (8), les zones cylindriques (7) et (8) sont réunies par un épaulement (11). L'épaulement (11) porte avantageusement une rainure (12) destinée à recevoir un joint d'étanchéité (13).

La zone conique (6) et la première zone cylindrique (7) forment la chambre de dissolution (24) de la cellule de dissolution.

Le bouchon (2) s'adapte à l'extrémité ouverte (5) du récipient (1) extérieurement à celui-ci par vissage, le récipient (1) étant muni sur sa surface extérieure d'une zone filetée (14).

Le bouchon (2) comporte des moyens de sortie du soluté constitués par un passage (15). Le passage (15) est avantageusement taraudé au voisinage de la surface supérieure (16) du bouchon (2) pour permettre l'adaptation aisée, au moyen d'un raccord, du conduit d'amenée du soluté à un appareil d'analyse.

Le bouchon (2) comporte également des moyens de passage (18) pour introduire dans la cellule au moins une sonde de mesure (56). Un logement (19) est prévu pour placer un joint d'étanchéité, par exemple, un joint torique pour assurer l'étanchéité entre le bouchon (2) et la sonde de mesure (56).

La cellule (1) est munie de moyens de filtration constitués par un filtre (20) maintenu entre le bouchon (2), qui est alors pourvu d'un bossage (21), et l'épaulement (11) du récipient (1). Un logement (22) est prévu dans le bossage (21) du bouchon (2) afin de placer un joint (23) pour assurer l'étanchéité entre le filtre (20) et le bouchon (2).

Le filtre (20) est ainsi placé sur le trajet du soluté entre la chambre de dissolution (24) et le passage (15) de sortie du soluté vers un appareil d'analyse.

Le filtre (20) est formé de microfibres de verre assemblées entre elles par une résine.

Le filtre (20) peut présenter une porosité voisine de 1 ».

Le filtre (20), selon le mode de réalisation représenté figure 1, a une hauteur faible vis-à-vis de son diamètre extérieur. On peut placer dans la cellule de dissolution représentée un filtre (20) d'une hauteur plus importante, le bouchon (2) sera alors moins engagé sur la zone filetée (14) située sur la surface extérieure du récipient (1).

La cellule de dissolution selon la figure 2 est un mode de réalisation de l'invention. La cellule comprend un récipient (1), analogue à celui du mode de réalisation selon la figure 1, formé d'une zone conique (6), d'une première (7) et d'une deuxième (8) zones cylindriques. Un conduit (9) d'entrée de liquide débouche au sommet de la zone conique (6) qui contient un garnissage (10) formé de billes de verre.

Comme décrit ci-avant, la première zone cylindrique (7) et la deuxième zone cylindrique (8) sont réunies par un épaulement (11), et la zone conique (6) et la première zone cylindrique (7) forment la chambre de dissolution (24) de la cellule de dissolution.

Selon le mode de réalisation représenté, le bouchon (2) s'adapte à l'extrémité ouverte (5) du récipient (1) intérieurement à celui-ci, en étant inséré dans la deuxième zone cylindrique (8).

Le bouchon (2) comporte, de l'extérieur à l'intérieur de la cellule de dissolution, un corps (25) d'une section sensiblement égale à celle de la deuxième zone cylindrique (8), une tête (26) d'une section inférieure à celle du corps (25) et une zone de collecte (27) formée par une gorge située entre le corps (25) et la tête (26) du bouchon (2).

Les moyens de sortie du soluté sont constitués par un conduit (15) traversant le corps (25) du bouchon (2) et débouchant par l'intermédiaire de la gorge (27) dans l'espace (28) situé entre la tête (26) et la paroi du récipient (1). Le conduit (15) est bien sûr taraudé au voisinage de la face supérieure (16) du bouchon (2) pour permettre l'adaptation d'un conduit au moyen d'un raccord.

Les moyens de filtration (3) sont constitués par un filtre tubulaire (20), sensiblement cylindrique, maintenu entre la tête (26) du bouchon (2) et l'épaulement (11) du récipient (1).

L'espace annulaire (29) situé entre le filtre (20) et la paroi du récipient (1) est en relation avec le conduit (15) par l'intermédiaire de l'espace (28) et de la zone collecte (27).

Le filtre tubulaire (20) permet de disposer d'une surface de filtration élevée d'une part et d'autre part de pouvoir faire varier, pour une cellule donnée la surface de filtration. En effet, en plaçant dans la cellule de dissolution, entre la tête (26) du bouchon (2) et l'épaulement (11) du récipient (1) des filtres (20) de hauteurs différentes, on peut faire varier la surface de filtration de la cellule de dissolution. Bien sûr, selon la hauteur du filtre (20), le bouchon (2) sera plus ou moins inséré dans la deuxième zone cylindrique (8).

Des joints toriques (13, 23) assurent l'étanchéité d'une part, entre le filtre (20) et l'épaulement (11) et, d'autre part, entre le filtre (20) et la tête (26) du bouchon (2).

Le bouchon (2) comporte, comme ci-avant des moyens de passage (18) pour introduire dans la cellule de dissolution au moins une sonde de mesure (56) et un logement (19) pour placer un joint destiné à assurer l'étanchéité entre le bouchon (2) et la sonde de mesure (56).

La cellule de dissolution selon ce mode de réalisation présente des moyens pour maintenir un échantillon solide. Ces moyens sont constitués par une nacelle (32), d'un type habituellement utilisé, maintenue par des encoches (33) réalisées dans la paroi de la première zone cylindrique (7) du récipient (1).

Le mode de réalisation de la cellule de dissolution représenté figure 3 est analogue à celui répresenté figure 2, cependant le bouchon (2) ne comporte pas de moyens de passage pour une sonde de mesure.

Selon ce mode de réalisation les moyens d'étanchéité entre le filtre (20) et l'épaulement (11) du récipient (1) et entre le filtre (20) et la tête (26) du bouchon (2) sont formés par des nervures annulaires (30,31) portées respectivement par l'épaulement (11) et la tête (26) du bouchon (2). Lors du montage, les nervures (30,31) pénètrent légèrement dans le filtre (20) et assurent ainsi l'étanchéité de la chambre de dissolution (24) vis-à-vis des particules de liant.

La cellule de dissolution selon le mode de réalisation représenté figure 4 comporte un filtre (20) tubulaire en forme de tronc de cône et le bouchon (2) ne présente pas de zone de collecte entre la tête (26) et le corps (25). Un filtre (20) en forme de tronc de cône permet encore d'augmenter la surface de filtration de la cellule de dissolution.

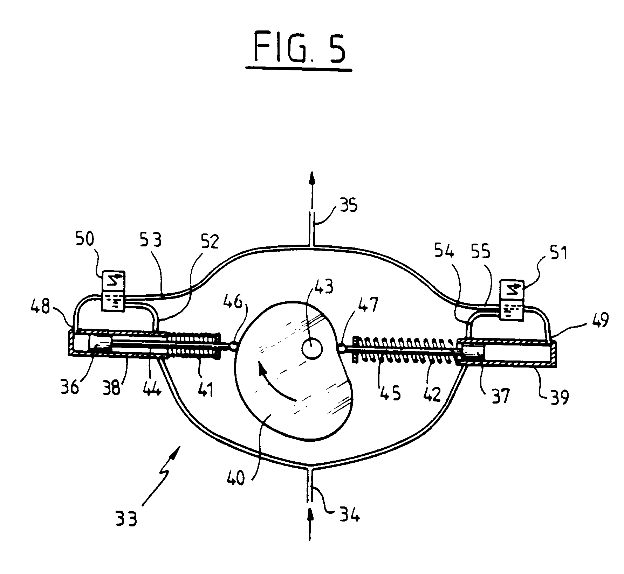

La figure 5 représente schématiquement les moyens de pompage (33) d'un appareil d'étude de la cinétique de dissolution d'un solide, objet de l'invention.

Ces moyens de pompage (33) sont reliés au moyen du conduit (34) à un réservoir d'alimentation de liquide et au moyen du conduit (35) au conduit (9) d'entrée du liquide dans la cellule de dissolution.

Les moyens de pompage (33) comportent deux pistons (36,37), montés en opposition, se déplaçant dans deux cylindres (38,39). Les pistons (36,37) sont animés d'un mouvement linéaire alternatif au moyen d'une came plate (40), possédant un profil approprié, à laquelle sont associés des moyens de rappel constitués par deux ressorts (41,42). Les pistons (36,37) sont montés de façon symétrique par rapport à l'axe (43) de rotation de la came (40).

Chaque tige (44,45) de piston (36,37) est guidée linéairement et est munie d'un dispositif d'anti-rotation. Les tiges (44,45) des pistons (36,37) s'appuient sur la came (40) par l'intermédiaire de galets (46,47). Le contact permanent entre les galets (46,47) et la came (40) est assuré par les ressorts (41,42).

L'axe (43) de la came (40) est entraîne en rotation, il est perpendiculaire à l'axe de déplacement des pistons (36,37). L'axe (43) peut être entraîné au moyen d'un moteur et d'une transmission par exemple par courroie ou par pignons. De préférence, afin d'éviter les jeux ou glissements, la came (40) est directement montée sur l'arbre de sortie d'un motoréducteur à vitesse lente, constante et connue avec précision.

Chaque cylindre (38,39) a sa sortie (48,49) reliée à une électrovanne (50,51) a trois voies et deux positions. Les sorties (52) et (54) des électrovannes (50,51) sont reliées entre elles et, par le conduit (34) sont en relation avec le réservoir d'alimentation de liquide, et les sorties (53) et (55) sont reliées entre elles et par le conduit (35) sont en relation avec la cellule de dissolution.

La manoeuvre des électrovannes (50,51) est commandée au moyens de contacts électriques activés à des positions angulaires très précises de la came (40).

Le mouvement des deux pistons (36,37) est déterminé par la forme de la came (40). Le profil de celle-ci est défini de façon telle que la somme instantanée des vitesses d'avance des pistons soit constante.

Le débit obtenu avec de tels moyens de pompage est parfaitement régulier, même pour de très faibles débits, et est indépendant de la pression de refoulement. De tels moyens de pompage sont également volumétriques, un tour de came délivrant deux volumes de cylindre, connaissant la vitesse de rotation de la came il est aisé de connaître avec précision le débit de liquide destiné à l'alimentation de la cellule de dissolution.

La cellule de dissolution pour solides et l'appareil de dissolution la comportant, objets de l'invention, présentent de nombreux avantages.

Ainsi la cellule de dissolution permet de disposer, grâce à la présence d'un filtre tubulaire d'une surface de filtration élevée ce qui est particulièrement intéressant pour limiter les inconvénients liés au colmatage du filtre par les particules de liant.

De plus, de par sa conception la cellule permet un montage et un démontage aisés. En outre, la possibilité d'introduire dans la chambre de dissolution des sondes de mesure permet de contrôler avec précision les conditions opératoires de la dissolution d'un produit solide.

L'appareil selon l'invention présente l'avantage de permettre des conditions d'écoulement de liquide dans la cellule particulièrement régulières, l'écoulement de liquide étant non pulsé et d'un débit volumique connu avec précision.

La cellule de dissolution et l'appareil de dissolution, objets de l'invention, sont particulièrement destinés à l'étude de cinétiques de dissolution de solides dans l'industrie pharmaceutique.

## Revendications

1. Cellule de dissolution pour solides, comprenant :
- un récipient (1) fermé à l'extrémité basse (4) et ouvert à l'extrémité haute (5) présentant intérieurement de bas en haut :
. une zone conique (6) divergente portant des moyens d'entrée de liquide (9) à son sommet, ladite zone contenant un garnissage (10) pour répartir le liquide,
. une première (7) et une deuxième (8) zones sensiblement cylindriques, la première zone (7) étant d'une section inférieure à celle de la deuxième zone (8), lesdites zones étant réunies par un épaulement (11),
. la zone conique (6) et la première zone cylindrique (7) formant la chambre de dissolution (24),
- un bouchon (2) destiné à s'adapter de façon étanche à l'extrémité ouverte (5) du récipient (1) et comportant des moyens de sortie (15) du soluté,
caractérisée en ce que l'on peut faire varier la position du bouchon (2) par rapport à l'extrémité ouverte du récipient (1), et en ce que la cellule comprend en outre des moyens de filtration constitués par un filtre tubulaire (20) maintenu entre le bouchon (2) et l'épaulement (11), et placé sur le trajet du soluté entre la chambre de dissolution (24) et les moyens de sortie (15) du soluté.

2. Cellule de dissolution selon la revendication 1, caractérisée en ce que le bouchon (2) comporte des moyens de passage (18) pour introduire au moins une sonde de mesure (56) dans la chambre de dissolution (24).

3. Cellule de dissolution selon l'une quelconque des revendications 1 et 2, caractérisée en ce que le bouchon (2) s'adapte à l'extrémité ouverte (5) du récipient (1), extérieurement au récipient (1).

4. Cellule de dissolution selon l'une des revendications 1 et 2, caractérisée en ce que le bouchon (2) s'adapte à l'extrémité ouverte du récipient (1), intérieurement au récipient.

5. Cellule de dissolution selon la revendication 4, caractérisée en ce que le bouchon (2) est inséré dans ledit récipient (1), et comporte de l'extérieur à l'intérieur de la cellule, un corps (25) d'une section sensiblement égale à celle de la deuxième zone (8) cylindrique et une tête (26) d'une section inférieure à celle du corps (25) et en ce que les moyens de sortie (15) du soluté sont constitués par un conduit traversant le corps (25) et débouchant dans l'espace (28) situé entre la tête (26) et la paroi du récipient (1).

6. Cellule de dissolution selon l'une quelconque des revendications précédentes, caractérisée en ce que le bouchon (2) comporte entre le corps (25) et la tête (26) une zone de collecte (27).

7. Cellule de dissolution selon l'une quelconque des revendications précédentes caractérisée en ce que le filtre tubulaire (20) est en microfibres de verre.

8. Cellule de dissolution selon l'une quelconque des revendications précédentes, caractérisée en ce qu'elle comporte des moyens d'étanchéité (13, 23) entre l'épaulement (11) et le filtre (20) et/ou entre le filtre (20) et le bouchon (2).

9. Cellule de dissolution selon l'une quelconque des revendications 1 à 8, caractérisée en ce qu'elle comporte des moyens (32) pour maintenir un échantillon de solide.

10. Appareil d'étude de la cinétique de dissolution d'un solide caractérisé en ce qu'il comporte au moins une cellule de dissolution selon l'une quelconque des revendications 1 à 9, à laquelle sont associés des moyens de pompage (33).

11. Appareil selon la revendication 10, caractérisé en ce que les moyens de pompage (33) comportent au moins deux pistons (36,37) montés en opposition déplacés par une came (40) tournant autour d'un axe (43) perpendiculaire à l'axe de déplacement des pistons (36,37) et des moyens de rappel (41, 42) associés à chaque piston.

## Claims

1. Dissolution cell for solids, comprising:
- a receptacle (1) closed at the bottom end (4) and open at the top end (5) and having on the inside, from bottom to top:
. a diverging conical zone (6) having inlet means (9) for liquid at its tip, the said zone containing a packing (10) for distributing the liquid,
. a first zone (7) and a second zone (8) which are essentially cylindrical, the first zone (7) being of a cross-section smaller than that of the second zone (8), the said zones being connected by a shoulder (11),
. the conical zone (6) and the first cylindrical zone (7) forming the dissolution chamber (24),
- a closure (2) intended to fit in a leakproof manner to the open end (5) of the receptacle (1) and comprising outlet means (15) for the solute,
characterized in that the position of the closure (2) can be varied relative to the open end of the receptacle (1), and in that the cell additionally comprises filtration means consisting of a tubular filter (20) held between the closure (2) and the shoulder (11) and placed in the pathway of the solute between the dissolution chamber (24) and the outlet means (15) for the solute.

2. Dissolution cell according to Claim 1, characterized in that the closure (2) comprises passageway means (18) for introducing at least one measurement probe (56) into the dissolution chamber (24).

3. Dissolution cell according to either one of Claims 1 and 2, characterized in that the closure (2) is fitted to the open end (5) of the receptacle (1), to the outside of the receptacle (1).

4. Dissolution cell according to either of Claims 1 and 2, characterized in that the closure (2) is fitted to the open end of the receptacle (1), to the inside of the receptacle.

5. Dissolution cell according to Claim 4, characterized in that the closure (2) is inserted into the said receptacle (1) and comprises, from the outside to the inside of the cell, a body (25) of a cross-section essentially equal to that of the second cylindrical zone (8) and a head (26) of a cross-section smaller than that of the body (25), and in that the outlet means (15) for the solute consist of a conduit passing through the body (25) and opening out into the space (28) situated between the head (26) and the wall of the receptacle (1).

6. Dissolution cell according to any one of the preceding claims, characterized in that the closure (2) comprises, between the body (25) and the head (26), a collecting zone (27).

7. Dissolution cell according to any one of the preceding claims, characterized in that the tubular filter (20) is made up of glass microfibres.

8. Dissolution cell according to any one of the preceding claims, characterized in that it comprises leakproofing means (13, 23) between the shoulder (11) and the filter (20) and/or between the filter (20) and the closure (2).

9. Dissolution cell according to any one of Claims 1 to 8, characterized in that it comprises means (32) for holding a sample of solid.

10. Apparatus for studying the kinetics of dissolution of a solid, characterized in that it comprises at least one dissolution cell according to any one of Claims 1 to 9, with which pumping means (33) are associated.

11. Apparatus according to Claim 10, characterized in that the pumping means (33) comprise at least two pistons (36, 37) mounted opposite each other and displaced by a cam (40) turning about an axis (43) perpendicular to the axis of displacement of the pistons (36, 37) and return means (41, 42) connected to each piston.

## Patentansprüche

1. Lösezelle für Feststoffe, die folgendes enthält:
- einen an der Unterseite (4) geschlossenen und an der Oberseite (5) offenen Behälter (1), welcher von unten nach oben gesehen folgendes aufweist:
. einen divergierenden konischen Bereich (6), der an seiner Spitze Mittel (9) für die Einfüllung einer Flüssigkeit aufweist, wobei dieser Bereich mit einem Futter (10) für die Verteilung der Flüssigkeit versehen ist;
. eine erste (7) und eine zweite (8) weitgehend zylindrische Zone, wobei die erste Zone (7) einen kleineren Querschnitt hat, als die zweite Zone (8), und die beiden Zonen durch einen Bund (11) miteinander verbunden sind;
. wobei der konische Bereich (6) und die erste zylindrische Zone (7) eine Lösungskammer (24) bilden,
- mit einem Verschluß (2), der leckdicht in das offene Ende (5) des Behälters (1) eingesetzt ist und Auslaßmittel (15) für den gelösten Stoff enthält,
**dadurch gekennzeichnet**, **daß**
die Position des Verschlusses (2) gegenüber dem offenen Ende des Behälters (1) verändert werden kann, und dadurch, daß die Zelle weiterhin Filtermittel aufweist, welche aus einem rohrförmigen Filter (20) bestehen, der zwischen dem Verschluß (2) und dem Bund (11) angebracht und in der Fließbahn des gelösten Stoffes zwischen der Lösungskammer (24) und den Auslaßmitteln (15) für den gelösten Stoffes angeordnet ist.

2. Lösezelle nach Anspruch 1,
**dadurch gekennzeichnet**, **daß**
der Verschluß (2) einen Durchgang (18) aufweist, um mindestens einen Meßfühler (56) in die Lösekammer (24) einführen zu können.

3. Lösezelle nach einem der Ansprüche 1 oder 2,
**dadurch gekennzeichnet**, **daß**
der Verschluß (2) an der Außenseite des Behälters (1) in das offene Ende (5) des Behälters (1) eingesetzt wird.

4. Lösezelle nach einem der Ansprüche 1 oder 2,
**dadurch gekennzeichnet**, **daß**
der Verschluß (2) an der Innenseite des Behälters in das offene Ende des Behälters (1) eingesetzt wird.

5. Lösezelle nach Anspruch 4,
**dadurch gekennzeichnet**, **daß**
der Verschluß (2) in den Behälter (1) eingeschoben wird und von der Innenseite zur Außenseite der Zelle einen Körper (25) mit einem Querschnitt aufweist, welcher weitgehend gleich dem Querschnitt des zweiten zylindrischen Bereiches (8) ist, sowie einen Kopf (26) mit einem Querschnitt, der kleiner ist, als der Querschnitt des Körpers (25), und dadurch, daß die Auslaßmittel (15) für den gelösten Stoff aus einer den Körper (25) durchquerenden Leitung bestehen, welche in den zwischen dem Kopf (26) und der Wand des Behälters (1) liegenden Raum (28) mündet.

6. Lösezelle nach einem der vorausgegangenen Ansprüche,
**dadurch gekennzeichnet**, **daß**
der Verschluß (2) zwischen dem Körper (25) und dem Kopf (26) einen Sammelbereich (27) aufweist.

7. Lösezelle nach einem der vorausgegangenen Ansprüche,
**dadurch gekennzeichnet**, **daß**
der rohrförmige Filter (20) aus Mikrofasern aus Glas besteht.

8. Lösezelle nach einem der vorausgegangenen Ansprüche,
**dadurch gekennzeichnet**, **daß**
sie zwischen dem Bund (11) und dem Filter (20) und/oder dem Filter (20) und dem Verschluß (2) Abdichtmittel (13, 23) aufweist.

9. Lösezelle nach einem der Ansprüche 1 bis 8,
**dadurch gekennzeichnet**, **daß**
sie Mittel (32) für die Aufnahme einer Probe des Feststoffes enthält.

10. Vorrichtung für die Untersuchung der Lösekinetik eines Feststoffes,
**dadurch gekennzeichnet**, **daß**
sie mindestens eine Lösezelle nach einem der Ansprüche 1 bis 9 aufweist, an die Pumpenmittel (33) angeschlossen sind.

11. Vorrichtung nach Anspruch 10,
**dadurch gekennzeichnet**, **daß**
die Pumpenmittel (33) mindestens zwei Kolben (36, 37) aufweisen, welche in gegenüberliegender Stellung getrennt durch eine Nocke (40) angeordnet sind und sich um eine Achse (43) drehen, welche senkrecht zur Bewegungsachse der Kolben (36, 37) verläuft, sowie mit den jeweiligen Kolben verbundene Rückzugsmittel (41, 42) enthält.
